# FASCICULE DE BREVET EUROPEEN

(11) **EP 3 269 409 B1**
(45) Date de publication et mention de la délivrance du brevet: **06.02.2019**
(21) Numéro de dépôt: 17180566.6
(22) Date de dépôt: 10.07.2017
(51) Int. Cl.: A61M 5/00, B01L 9/06, B65D 21/02, B65D 25/00, B65B 3/00

(54) **NEST À SERINGUES**
NEST MIT SPRITZEN
SYRINGE NEST

(30) Priorité: 11.07.2016 BE 201605580
(43) Date de publication de la demande: 17.01.2018
(73) Titulaire: Sybermat SA, 1420 Braine-l'Alleud (BE)
(72) Inventeur: MANCIONE, Antonio, 59264 ONNAING (FR)
(74) Mandataire: Gevers Patents

(56) Documents cités:
- EP-A1- 1 138 390
- EP-A1- 2 476 448
- EP-A1- 2 865 400
- WO-A2-2013/181552
- US-A- 5 823 363
- US-A1- 2015 114 853

## Description

La présente invention se rapporte à un nest à seringues.

Les nests sont des présentoirs pour seringues, généralement en polypropylène. Ils comprennent une plaque basale munie de tubes de retenue qui s'étendent perpendiculairement à la plaque basale sur une hauteur prédéterminée et dans lesquels les seringues sont enfoncées dans une position de maintien dans le nest, lesdits tubes présentant une première extrémité, une zone centrale et une deuxième extrémité à l'opposé de ladite première extrémité. A l'une des extrémités des seringues sera fixée à un moment donné une aiguille d'injection, tandis que l'autre extrémité des seringues est pourvue d'une collerette d'un diamètre supérieur à celui du corps de seringue.Lorsque les seringues sont enfilées dans les tubes, elles sont retenues par les collerettes. Cette façon de conditionner les seringues est largement répandue. Lorsqu'une société pharmaceutique souhaite conditionner un produit dans une seringue, en particulier dans une seringue en verre, elle s'adresse à des fabricants de seringues et elles sont livrées en présentoirs plus communément appelés nests. Généralement les nests sont livrés dans un boîtier de protection qui protège les seringues de la casse, les seringues étant disposées parallèlement et maintenues substantiellement écartées l'une de l'autre justement grâce auxdits nests.

Les boîtiers de protection présentent généralement un rebord interne sur lequel vient reposer la plaque basale du nest.

Les seringues sont remplies du produit qu'elles sont censées contenir par l'extrémité munie de la collerette et elles présentent un bouchon placé sur les aiguilles tandis que le poussoir du piston n'est quant à lui ajouté qu'à la toute fin du conditionnement, juste avant l'emballage.

Malheureusement, certains nests actuels comprennent des tubes qui ne permettent pas une introduction et une extraction des seringues qui soient aisées. En effet, lors de ces étapes, des pinces robotisées présentent les seringues séparées les unes des autres d'une distance appropriée de manière que, lorsque les seringues sont lâchées par la pince, celles-ci s'introduisent correctement dans les tubes du nest. Ce procédé est appelé le nestage des seringues. L'étape inverse qui consiste à extraire les seringues des tubes au moyen d'une pince prévue à cet effet est appelée le dénestage. Ces étapes sont délicates lors du procédé de conditionnement des seringues notamment parce que les bouchons présents sur les aiguilles des seringues ou le corps même des seringues se coincent aux extrémités des tubes du nest par lesquelles ils doivent être passés. En effet, lors des procédés de nestage ou de dénestage, il est fréquent qu'un bouchon heurte l'une des extrémités des tubes du nest. L'accrochage entre le bouchon et le tube du nest peut provoquer l'arrachage du bouchon ou provoquer le déséquilibre ou le soulèvement du nest. Il s'ensuit alors un arrêt du procédé de conditionnement des seringues voire même la casse d'une ou de plusieurs seringues en verre. Ces désagréments ne sont pas souhaitables car, d'une part, ils nécessitent des arrêts de production provoquant ainsi des pertes économiques considérables. D'autre part, une seringue cassée peut donner lieu à la présence d'éclats de verre minuscules, voire microscopiques, dans les autres seringues. Le verre cassé est un problème majeur en raison du risque qu'il présente de provoquer de sérieux troubles chez le patient auquel cette fraction microscopique de verre aurait été injectée, par exemple lors de l'administration d'un vaccin. L'état de la technique en vue de la présente invention est représenté par les documents US5823363 et EP2476448.

Pour pallier ces inconvénients, le document EP1138390 divulgue un type de nest pouvant transporter des seringues dans lequel au moins un tube présente une paroi interne qui, dans la zone centrale, est en contact au moins partiel avec un corps de seringue dans la position de maintien et qui, à au moins une des première et deuxième extrémités, n'est pas en contact avec le corps de seringue en position de maintien.

Un nest à seringues selon ce document antérieur permet de limiter les risques d'accrochage entre les seringues, en particulier les risques d'arrachage de leur bouchon, et les tubes du nest, ceci pouvant survenir lors des procédés de nestage et/ou de dénestage.

En effet, selon le document EP1138390, la paroi interne permet de maintenir le corps de seringue verticalement lors du transport. De cette manière, lorsque toutes les seringues sont maintenues le plus verticalement possible dans le nest à seringues, les mouvements latéraux de celles-ci sont minimisés et les contacts entre les parties libres des seringues, c'est-à-dire les parties situées sous le nest, sont réduits en permettant notamment d'éviter la casse des seringues. De plus, grâce au maintien des seringues dans la zone centrale des tubes par la paroi interne des tubes ou au moins une partie de celle-ci, les seringues restent bien positionnées et centrées dans les tubes. Ce centrage des seringues dans les tubes est important car les pinces robotisées permettant le nestage ou le dénestage sont calibrées de manière à être adaptées à l'intervalle particulier entre les tubes du nest. De ce fait, si les seringues ne sont pas correctement centrées dans les tubes, l'intervalle entre les seringues peut ne plus correspondre à l'intervalle défini pour la pince robotisée. Par conséquent, un mauvais centrage des seringues dans les tubes du nest provoque principalement des problèmes lors du procédé de dénestage en raison d'un manque de complémentarité entre l'intervalle prédéfini de la pince robotisée et l'intervalle entre les seringues. La pince robotisée n'est dès lors pas capable de saisir correctement les seringues pour les extraire du nest lors du dénestage.

Par ailleurs, la paroi interne du tube selon ce document antérieur est telle qu'à au moins une des première et deuxième extrémités, le corps de seringue en position de maintien n'est pas en contact avec la paroi interne. De plus, ladite paroi interne n'est pas en contact avec le corps de seringue en position de maintien aux deux extrémités des tubes. Cela permet, lors du nestage, que le corps de seringue, et en particulier le bouchon placé au niveau de l'aiguille, ne rencontre aucun obstacle et ne coure donc aucun danger de coincement avec le tube. En effet, le bouchon commence à être guidé par la paroi interne du tube alors qu'il est déjà en partie à l'intérieur de celui-ci. Il en est de même lors du dénestage.

En fait, l'espace libre permettant un passage du bouchon placé sur l'aiguille et du corps de seringue à travers le tube s'amincit depuis de préférence chaque extrémité du tube vers la zone centrale de celui-ci. De cette manière, le diamètre interne de cet espace libre susdit du tube à l'extrémité tournée vers la pince robotisée et à l'extrémité à l'opposé de la pince robotisée, est supérieur au diamètre de l'espace libre dans la zone centrale du tube. Il en résulte une introduction plus aisée des seringues lors du procédé de nestage et une extraction facilitée lors du dénestage des seringues.

Etant donné que le tube ne présente plus d'arrêté vive à l'extrémité tournée vers la pince robotisée, le bouchon placé sur l'aiguille de la seringue n'accroche plus le bord du tube et pénètre de ce fait plus facilement dans le tube sans coincement.

Par ailleurs, lors d'un procédé de dénestage, le tube présente un espace libre de diamètre interne plus important à son extrémité à l'opposé de la pince robotisée de manière que, lorsque les seringues sont extraites des tubes, le bouchon placé sur la seringue ne soit pas arraché ou ne provoque pas le soulèvement du nest.

Par conséquent, grâce à un nest selon ce document antérieur, les arrêts de production ou les problèmes de déséquilibre des nests sont nettement réduits et le risque de casse des seringues est minimisé grâce au bon maintien des seringues.

Malheureusement, les nests à seringues connus de l'état de la technique, dont le nest à seringues selon le document EP1138390, présentent plusieurs inconvénients.

D'une part, il s'avère, qu'avec les nests à seringues actuels, des problèmes de rigidité sont rencontrés. En effet, lorsque les tubes des nests actuels sont occupés par des seringues en verre, par exemple remplies de leur contenu, le poids total que doit supporter le nest est important et peut donner lieu à des déformations de ce dernier. Par ailleurs, le nest à seringues lorsqu'il est disposé dans un boîtier de protection, ne repose que sur un rebord interne du boitier prévu à cet effet, la partie centrale du nest n'étant alors pas soutenue. Le nest peut alors se déformer sous le poids des seringues provoquant ainsi une modification de la forme du nest qui devient convexe par rapport au boîtier de protection. Par conséquent, les intervalles entre les seringues diminuent. De ce fait, étant donné que les intervalles entre les seringues sont modifiés et ne sont plus contrôlés, des problèmes lors du dénestage peuvent survenir à cause d'un manque de complémentarité entre les intervalles entre les seringues et les intervalles définis dans (au niveau de) la pince robotisée.

D'autre part, des problèmes de contamination, par exemple de contamination bactérienne, sont souvent rencontrés lors du procédé de conditionnement des seringues avec les nest actuels. En effet, avec de tels nests, de l'eau peut se condenser et stagner sur la plaque basale ou sur la surface (zone) présente entre les tubes de nests, notamment suite à une étape de stérilisation des nests par injection de vapeur. La contamination du contenu des seringues est un problème majeur vu l'impact que cela engendre sur la qualité et l'efficacité des produits, mais également en raison du risque qu'une telle contamination présente de provoquer de sérieux troubles chez le patient.

L'invention a pour but de pallier les inconvénients de l'état de la technique en procurant un nest à seringues qui présente une rigidité améliorée et qui diminue les risques de contamination, par exemple de contamination bactérienne, lors du procédé de conditionnement des seringues, notamment suite à une stérilisation par injection de vapeur. Pour résoudre ce problème, il est prévu selon l'invention, un nest à seringue selon la revendication 1.

Il a été montré qu'un nest à seringues selon l'invention permet d'améliorer considérablement la rigidité du nest à seringue. Comme indiqué plus haut, lorsque les tubes du nest à seringues sont occupés par des seringues en verre, par exemple remplies de leur contenu, le poids total que doit supporter le nest est important. L'amélioration de la rigidité du nest à seringues selon l'invention est dès lors avantageuse car elle permet de réduire la déformation du nest provoquée par le poids des seringues, ce qui permet de limiter davantage les problèmes techniques lors des procédés de nestage et de dénestage. En outre, un nest à seringues selon l'invention, dont chacun des tubes présente une paroi externe étant en contact avec les parois externes des tubes voisins le long d'une ligne de contact parallèle à l'axe longitudinal des tubes, présente un risque de contamination du contenu des seringues qui est fortement diminué. En effet, la paroi externe de chacun des tubes est en contact avec les parois externes des tubes voisins de telle façon qu'entre les tubes voisins, en particulier entre trois tubes voisins, un évidement est présent. De cette manière, la plaque basale du nest selon l'invention est présente uniquement sur le pourtour du nest, notamment pour prendre appui sur un boitier de protection, et n'est plus présente entre les tubes du nest comme c'est le cas avec les nests actuels. La présence d'évidements délimités par les parois externes des tubes de nest permet d'éviter l'accumulation et la stagnation d'eau sur le nest à seringues et diminue par conséquent le risque de contaminer le contenu des seringues. Ainsi, le nest à seringues selon l'invention autorise une stérilisation à la vapeur tout en laissant le passage libre de la vapeur et ce grâce à la présence des évidements définis par les parois externes de tubes voisins du nest selon l'invention, chacun desdits tubes dudit nest présentant une paroi externe étant en contact avec les parois externes des tubes voisins le long d'une ligne de contact parallèle à l'axe longitudinal desdits tubes.

Les nervures présentes sur la paroi interne du tube permettent en outre d'améliorer davantage le maintien des seringues dans les tubes du nest.

Dans une forme de réalisation particulière du nest à seringues selon la présente invention, lesdites au moins trois nervures s'étendent de ladite première extrémité à ladite deuxième extrémité dudit tube.

Avantageusement, les nervures présentent perpendiculairement à l'axe longitudinal dudit tube, une première épaisseur prédéterminée e1 à la première extrémité, une deuxième épaisseur prédéterminée e2 à la deuxième extrémité et une troisième épaisseur prédéterminée e3 au niveau de la zone centrale, de façon que le rapport e1/e3 et/ou le rapport e2/e3 soit inférieur à 1.

Dans cette forme de réalisation particulière, l'épaisseur des nervures au niveau d'au moins une des deux extrémités du tube est inférieure à l'épaisseur des nervures au niveau de la zone centrale du tube. Il en résulte une amélioration de l'introduction et/ou de l'extraction des seringues dans/hors des tubes lors du procédé de nestage ou du procédé de dénestage. Les nervures susdites servent de moyens de guidage et de centrage des seringues à l'intérieur des tubes.

Par ailleurs, la présence de nervures sur la paroi interne du tube permet également de réduire la quantité de matière nécessaire à la réalisation du nest à seringues.

De préférence, le nest à seringues est mis en forme par moulage par injection d'un polymère, de préférence d'un polypropylène.

Le nest suivant l'invention comprend avantageusement en outre des tubes présentant une paroi externe, la paroi externe de chaque tube du nest étant en contact avec la paroi externe des tubes voisins le long d'une ligne de contact parallèle à l'axe longitudinal desdits tubes.

Avantageusement, le nest à seringues selon l'invention présente une plaque basale comprenant en outre deux orifices de passage disposés de part et d'autre de ladite plaque.

Dans cette forme de réalisation, le nest comprend un orifice sur deux côtés parallèles afin de pouvoir y introduire les doigts, ce qui permet la préhension du nest.

De préférence, la plaque basale présente, en périphérie d'au moins un desdits orifices de passage, une saillie s'étendant parallèlement auxdits tubes sur une hauteur prédéterminée.

Avantageusement, ladite hauteur de ladite saillie est égale ou supérieure à ladite hauteur prédéterminée desdits tubes.

La hauteur de la saille qui est supérieure à la hauteur des tubes du nest selon la présente invention permet en outre de superposer des nests de manière stable.

De préférence, la partie libre de la saillie présente en périphérie des orifices de passage présente un diamètre inférieur au diamètre desdits orifices. De cette manière, lorsque deux nests sont empilés l'un sur l'autre, la partie libre de la saillie du nest sous-jacent peut pénétrer dans l'orifice du nest superposé. La stabilité de l'empilage des nests est alors améliorée.

D'autres formes de réalisation du dispositif suivant l'invention sont indiquées dans les revendications annexées.

L'invention a aussi pour objet un empilage de nests à seringues selon la présente invention, comprenant un premier nest à seringues sous-jacent et au moins un deuxième nest à seringues superposé au nest à seringues sous-jacent.

D'autres formes de réalisation de l'empilage de nests à seringues suivant l'invention sont indiquées dans les revendications annexées.

D'autres caractéristiques, détails et avantages de l'invention ressortiront de la description donnée ci-après, à titre non limitatif et en faisant référence aux dessins annexés.
La figure 1 est une vue en perspective d'un nest à seringues selon l'invention.
La figure 2 est une vue en plan du dessus d'un nest à seringues selon l'invention.
La figure 3 est une vue latérale d'un nest à seringues selon l'invention.
La figure 4 est une vue d'en haut d'une variante du nest à seringues selon l'invention.
La figure 5 est une section selon l'axe V-V d'un tube du nest présenté à la figure 4.
La figure 6 est un empilage de deux nest à seringues selon la présente invention.

Sur les figures, les éléments identiques ou analogues portent les mêmes références.

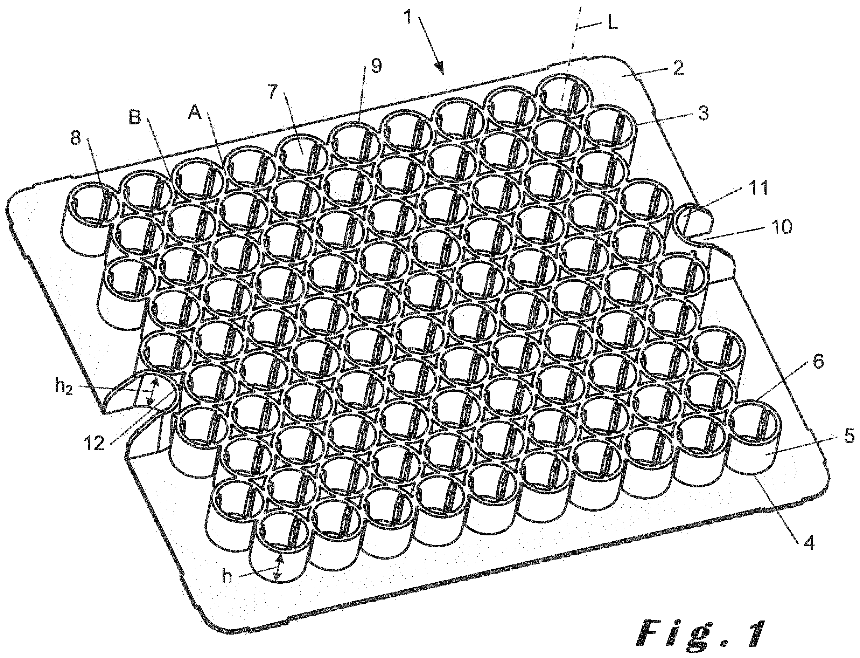

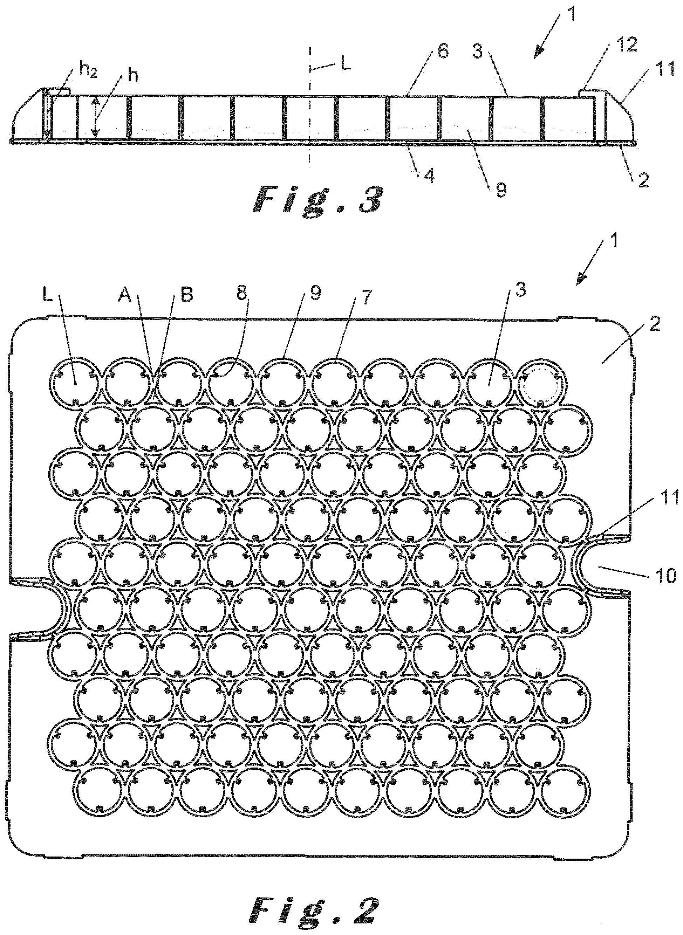

Les figures 1 et 2 illustrent un nest à seringues 1 comprenant une plaque basale 2 munie de tubes de retenue 3 qui s'étendent perpendiculairement à la plaque basale 2 sur une hauteur h prédéterminée et dans lesquels les seringues sont enfoncées lorsqu'elles sont dans une position de maintien dans le nest. Les tubes 3 présentent une première extrémité 4, une zone centrale 5 et une deuxième extrémité 6 à l'opposé de la première extrémité 4.

Dans le nest présenté sur la figure 1, les tubes 3 présentent une paroi interne 7 qui, dans la zone centrale 5, est en contact au moins partiel avec un corps de seringue (non représenté) dans ladite position de maintien et qui, aux première 4 et deuxième 6 extrémités, n'est pas en contact avec le corps de seringue en position de maintien. Sur les figures 1 et 2, les tubes 3 comprennent trois nervures 8 s'étendant parallèlement à un axe longitudinal L des tubes 3, sur toute la hauteur h du tube 3, de la première extrémité 4 à la deuxième extrémité 6 du tube.

Les tubes sur les figure 1 et 2 présentent une paroi externe 9, la paroi externe de chaque tube du nest étant en contact avec les parois externes des tubes voisins le long d'une ligne de contact A parallèle à l'axe longitudinal L desdits tubes 3. De cette manière, le nest présente un réseau de tubes 3 interconnectés les uns aux autres par leurs parois externes 9.

De préférence, il existe des zones B entre les lignes de contacts A au niveau desquelles la plaque basale 2 est percée de manière à éviter l'accumulation de poussière ou d'autres contaminants entre les tubes 3 du nest 1. Il s'agit en effet de zones B généralement difficilement accessibles dont le nettoyage n'est pas aisé, des zones B évidées selon l'invention permettant avantageusement d'éviter l'accumulation des poussières.

Sur les figures 1 et 2, la plaque basale 2 comprend en outre deux orifices de passage 10 disposés de part et d'autre de ladite plaque 2. La plaque basale présente en périphérie des orifices de passage 10 une saillie 11 s'étendant parallèlement aux tubes 3 sur une hauteur prédéterminée h2. Les orifices de passages 10 entourés d'une saillie 11 sont présents sur deux côtés parallèles du nest afin de pouvoir y introduire les doigts, ce qui permet la préhension du nest. Sur la figure 3 qui représente une vue latérale d'une forme de réalisation du nest à seringues 1 selon la présente invention, on peut voir que la hauteur h2 de la saillie 11 est supérieure à la hauteur h des tubes 3.

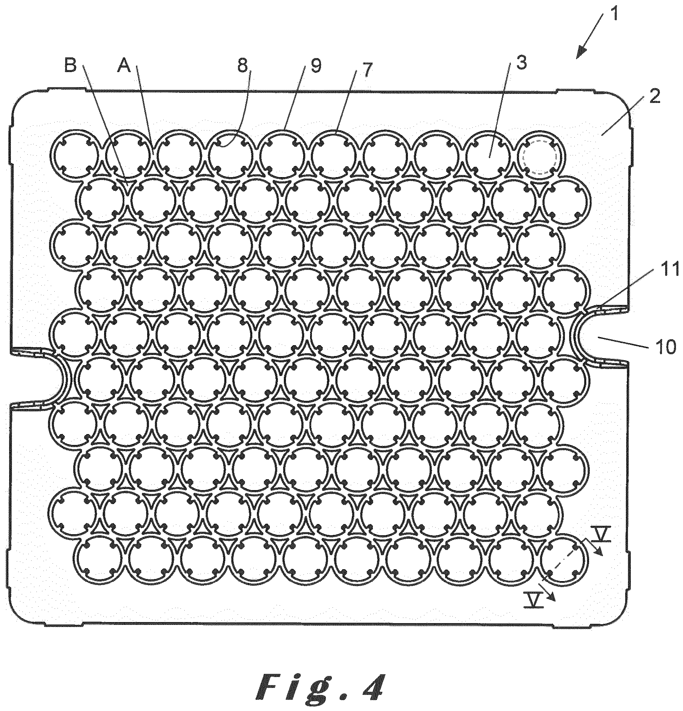

La figure 4 est une variante du nest à seringue selon la présente invention dans lequel les tubes 3 comprennent quatre nervures 8 s'étendant parallèlement à un axe longitudinal L des tubes 3, sur toute la hauteur h du tube 3, de ladite première extrémité 4 à ladite deuxième extrémité 6 dudit tube.

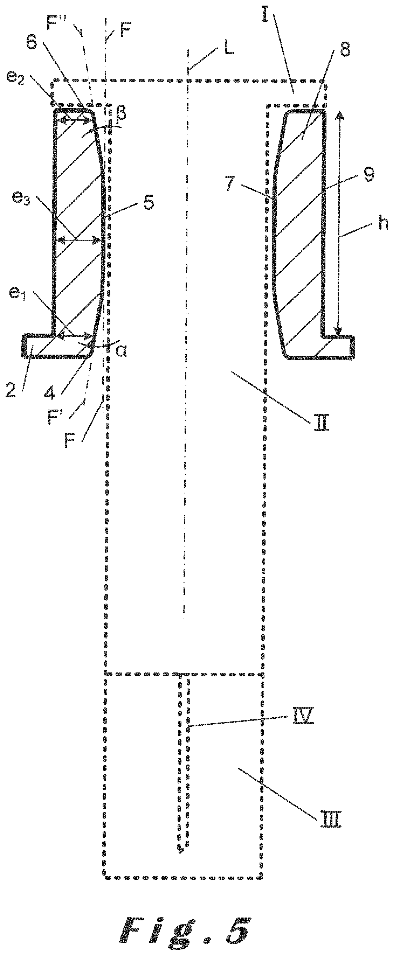

La figure 5 illustre une coupe axiale selon l'axe V-V du tube 3 présenté sur la figure 4. On peut voir sur la figure 5 que les nervures 8 présentent, perpendiculairement à l'axe longitudinal L du tube 3, une première épaisseur e1 à la première extrémité 4, une deuxième épaisseur e2 à la deuxième extrémité 6 et une troisième épaisseur e3 au niveau de la zone centrale 5, de façon que le rapport e1/e3 et le rapport e2/e3 soient inférieurs à 1.

La collerette I de la seringue vient reposer sur la deuxième extrémité 6 du tube 3 qui de cette manière retient verticalement la seringue dans le nest. Le corps II de seringue est quant à lui au moins partiellement en contact avec les nervures 8 au niveau de la zone centrale 5 du tube 3. Dans sa position de maintien la seringue est ainsi positionnée de manière à éviter des mouvements latéraux. Aux première 4 et deuxième 6 extrémités du tube, le corps II de la seringue n'est pas en contact avec les nervures 8. On peut voir sur la figure 5 qu'un bouchon III présentant un diamètre sensiblement égal au diamètre du corps II de la seringue est placé sur l'aiguille IV. La section illustrée à la figure 5 permet de mettre en évidence le profil biseauté des nervures 8 aux extrémités 4 et 6 du tube 3 servant alors de moyens de guidage et de centrage des seringues à l'intérieur des tubes. Par conséquent le passage du corps de seringue II et du bouchon III est plus aisé au niveau des extrémités 4 et 6 du tube 3.

A la figure 5 sont illustrées, sur la paroi interne du tube, une première droite F tangente à la zone centrale 5, une deuxième droite F' tangente à la première extrémité 4 et une troisième droite F" tangente à la deuxième extrémité 6. De préférence, le profil biseauté des nervures 8 est alors caractérisé au niveau de la première extrémité 4 par un angle α, mesuré entre la droite F et la droite F', compris entre 5 ° et 20 °, de préférence compris entre 10 ° et 15 ° et au niveau de la deuxième extrémité 6 par un angle β, mesuré entre la droite F et la droite F", compris entre 5 ° et 20 °, de préférence compris entre 10 ° et 15 °. Sur la figure 5, les angles α et β sont identiques mais on peut imaginer que les valeurs des angles α et β soient différentes.

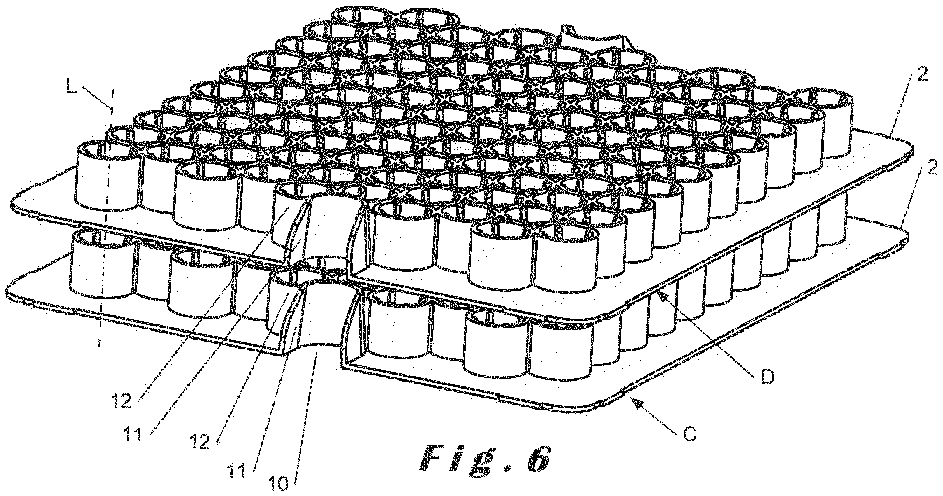

La figure 6 représente un empilage de nests à seringues selon l'invention, comprenant un premier nest à seringues sous-jacent C et un deuxième nest à seringues superposé C au nest à seringues sous-jacent C. Lorsque les deux nests à seringues C et D sont empilés l'un sur l'autre, l'extrémité libre 12 de la saillie 11 du nest sous-jacent C vient s'introduire dans l'orifice 10 du nest superposé D de manière à forme un ensemble stable.

Il est bien entendu que la présente invention n'est en aucune façon limitée aux formes de réalisations décrites ci-dessus et que bien des modifications peuvent y être apportées sans sortir du cadre des revendications annexées.

## Revendications

1. Nest à seringues (1) comprenant une plaque basale (2) munie de tubes de retenue (3) qui s'étendent perpendiculairement à la plaque basale (2) sur une hauteur (h) prédéterminée et dans lesquels les seringues sont enfoncées dans une position de maintien dans le nest (1),
(i) lesdits tubes (3) présentant une première extrémité (4), une zone centrale (5) et une deuxième extrémité (6) à l'opposé de ladite première extrémité (4),
(ii) au moins un desdits tubes (3) présentant une paroi interne (7) qui comprend au moins trois nervures (8) s'étendant parallèlement à un axe longitudinal (L) dudit tube (3), sur au moins une partie de la hauteur (h) prédéterminée dudit tube (3) et qui, dans la zone centrale (5), est en contact au moins partiel avec un corps de seringue dans ladite position de maintien et qui, à au moins une desdites première (4) et deuxième (6) extrémités, n'est pas en contact avec le corps de seringue en position de maintien,
le corps de seringue étant au moins partiellement en contact avec les nervures (8) au niveau de la zone centrale (5) du tube (3), le corps de la seringue n'étant pas en contact avec les nervures (8) aux première (4) et deuxième (6) extrémités du tube,
dans lequel chacun desdits tubes (3) dudit nest (1) présente une paroi externe (9) étant en contact avec les parois externes (9) des tubes (3) voisins le long d'une ligne de contact (A) parallèle à l'axe longitudinal (L) desdits tubes (3).

2. Nest à seringues (1) selon la revendication 1, dans lequel ladite paroi interne (7), auxdites première (4) et deuxième (6) extrémités, n'est pas en contact avec le corps de seringue en position de maintien.

3. Nest à seringues (1) selon l'une quelconque des revendications précédentes, dans lequel lesdites au moins trois nervures (8) s'étendent de ladite première extrémité (4) à ladite deuxième extrémité (6) dudit tube.

4. Nest à seringues selon l'une quelconque des revendications précédentes, dans lequel lesdites au moins trois nervures présentent, perpendiculairement à l'axe longitudinal (L) dudit tube (3), une première épaisseur prédéterminée (e1) à la première extrémité (4), une deuxième épaisseur prédéterminée (e2) à la deuxième extrémité (6) et une troisième épaisseur prédéterminée (e3) au niveau de la zone centrale (5), de façon que le rapport e1/e3 et/ou le rapport e2/e3 soit inférieur à 1.

5. Nest à seringues selon l'une quelconque des revendications précédentes, dans lequel la plaque basale (2) comprend en outre deux orifices de passage (10) disposés de part et d'autre de ladite plaque (2).

6. Nest à seringues selon la revendication 5, dans lequel la plaque basale présente en périphérie d'au moins un desdits orifices de passage (10) une saillie (11) s'étendant parallèlement auxdits tubes (3) sur une hauteur prédéterminée (h2).

7. Nest à seringues selon la revendication 6, dans lequel ladite hauteur (h2) de ladite saillie (11) est égale ou supérieure à ladite hauteur (h) desdits tubes (3).

8. Empilage de nests à seringues selon l'une quelconque des revendications 1 à 7, comprenant un premier nest à seringues sous-jacent et au moins un deuxième nest à seringues superposé au nest à seringues sous-jacent.

## Patentansprüche

1. Nest mit Spritzen (1), umfassend eine Basalplatte (2), die mit Rückhalterohren (3) versehen ist, die sich senkrecht zur Basalplatte (2) auf einer vorbestimmten Höhe (h) erstrecken und in denen die Spritzen in einer Halteposition in das Nest (1) eingetrieben sind,
(i) wobei die Rohre (3) ein erstes Ende (4), eine zentrale Zone (5) und ein zweites Ende (6), das dem ersten Ende (4) gegenüberliegt, aufweist,
(ii) wobei zumindest eines der Rohre (3) eine Innenwand (7) aufweist, die zumindest drei Rippen (8) umfasst, die sich parallel zu einer Längsachse (L) des Rohrs (3) auf zumindest einem Teil der vorbestimmten Höhe (h) des Rohrs (3) erstrecken, und die in der zentralen Zone (5) in zumindest teilweisem Kontakt mit einem Spritzenkörper in der Halteposition steht und die an zumindest einem des ersten (4) und zweiten (6) Endes nicht in Kontakt mit dem Spritzenkörper in der Halteposition steht,
wobei der Spritzenkörper zumindest teilweise in Kontakt mit den Rippen (8) an der zentralen Zone (5) des Rohrs (3) steht, wobei der Spritzenkörper nicht in Kontakt mit den Rippen (8) an dem ersten (4) und zweiten (6) Ende des Rohrs steht,
wobei jedes der Rohre (3) des Nests (1) eine Außenwand (9) aufweist, die mit den Außenwänden (9) der benachbarten Rohre (3) entlang einer zur Längsachse (L) der Rohre (3) parallelen Kontaktlinie (A) in Kontakt steht.

2. Nest mit Spritzen (1) nach Anspruch 1, wobei die Innenwand (7) an dem ersten (4) und zweiten (6) Ende nicht in Kontakt mit dem Spritzenkörper in der Halteposition steht.

3. Nest mit Spritzen (1) nach einem der vorstehenden Ansprüche, wobei sich die zumindest drei Rippen (8) vom ersten Ende (4) zum zweiten Ende (6) des Rohrs erstrecken.

4. Nest mit Spritzen nach einem der vorstehenden Ansprüche, wobei die zumindest drei Rippen senkrecht zur Längsachse (L) des Rohrs (3) eine erste vorbestimmte Dicke (e1) an dem ersten Ende (4), eine zweite vorbestimmte Dicke (e2) an dem zweiten Ende (6) und eine dritte vorbestimmte Dicke (e3) an der zentralen Zone (5) aufweisen, sodass das Verhältnis e1/e3 und/oder das Verhältnis e2/e3 weniger als 1 beträgt.

5. Nest mit Spritzen nach einem der vorstehenden Ansprüche, wobei die Basalplatte (2) weiter zwei Durchgangsöffnungen (10) umfasst, die beiderseits der Platte (2) angeordnet sind.

6. Nest mit Spritzen nach Anspruch 5, wobei die Basalplatte an der Peripherie zumindest einer der Durchgangsöffnungen (10) einen Vorsprung (11) aufweist, der sich parallel zu den Rohren (3) auf einer vorbestimmten Höhe (h2) erstreckt.

7. Nest mit Spritzen nach Anspruch 6, wobei die Höhe (h2) des Vorsprungs (11) gleich der oder größer als die Höhe (h) der Rohre (3) ist.

8. Stapel von Nestern mit Spritzen nach einem der Ansprüche 1 bis 7, umfassend ein erstes unterliegendes Nest mit Spritzen und zumindest ein zweites Nest mit Spritzen, das dem unterliegenden Nest mit Spritzen überlagert ist.

## Claims

1. Syringe nest (1) comprising a basal plate (2) provided with retaining tubes (3) which extend perpendicularly to the basal plate (2) on a predetermined height (h) and wherein the syringes are pushed into a maintaining position in the nest (1),
(i) said tubes (3) having a first end (4), a central zone (5) and a second end (6) opposite said first end (4),
(ii) at least one of said tubes (3) having an inner wall (7) which comprises at least three ribs (8) extending parallel to a longitudinal axis (L) of said tube (3), on at least one portion of the predetermined height (h) of said tube (3) and which, in the central zone (5), is in contact at least partially with a syringe body in said maintaining position and which, at at least one of said first (4) and second (6) ends, is not in contact with the syringe body in the maintaining position,
the syringe body being at least partially in contact with the ribs (8) on the central zone (5) of the tube (3), the body of the syringe not being in contact with the ribs (8) at the first (4) and second (6) ends of the tube,
wherein each of said tubes (3) of said nest (1) has an outer wall (9) being in contact with the outer walls (9) of the adjacent tubes (3) along a contact line (A) parallel to the longitudinal axis (L) of said tubes (3).

2. Syringe nest (1) according to claim 1, wherein said inner wall (7), at said first (4) and second (6) ends, is not in contact with the syringe body in the maintaining position.

3. Syringe nest (1) according to any one of the preceding claim, wherein said at least three ribs (8) extend from said first end (4) to said second end (6) of said tube.

4. Syringe nest according to any one of the preceding claims, wherein said at least three ribs have, perpendicular to the longitudinal axis (L) of said tube (3), a first determined thickness (e1) at the first end (4), a second determined thickness (e2) at the second end (6) and a third determined thickness (e3) at the level of the central zone (5), such that the ratio e1/e3 and/or the ratio e2/e3 is less than 1.

5. Syringe nest according to any one of the preceding claims, wherein the basal plate (2) further comprises two passage orifices (10) arranged on either side of said plate (2).

6. Syringe nest according to claim 5, wherein the basal plate has at the periphery of at least one of said passage orifices (10), a protrusion (11) extending parallel to said tubes (3) on a predetermined height (h2).

7. Syringe nest according to claim 6, wherein said height (h2) of said protrusion (11) is greater than or equal to said height (h) of said tubes (3).

8. Stack of syringe nests according to any one of claims 1 to 7, comprising a first underlying syringe nest and at least one second syringe nest superposed on the underlying syringe nest.
